Europäisches Patentamt

European Patent Office

Office européen des brevets

(19)

(11) Publication number: **0 190 049 B1**

(12) **EUROPEAN PATENT SPECIFICATION**

(45) Date of publication of patent specification: **05.08.92**    (51) Int. Cl.5: **C12N 5/12, C12P 21/08**

(21) Application number: **86300639.1**

(22) Date of filing: **30.01.86**

(54) **Method for selecting hybridomas producing antibodies specific to inaccessible cell surface antigens.**

(30) Priority: **30.01.85 CA 473176**

(43) Date of publication of application:
**06.08.86 Bulletin 86/32**

(45) Publication of the grant of the patent:
**05.08.92 Bulletin 92/32**

(84) Designated Contracting States:
**AT BE CH DE FR GB IT LI LU NL SE**

(56) References cited:

**JOURNAL OF CELLULAR BIOCHEMISTRY, Supplement 9A, "UCLA Symposia on Mulecular & Cellular Biology", Abstracts, 14th Annual Meetings, 12th January - 6th February 1985, page 64, no. 0151, Alan R. Liss, Inc., New York, US;N.KARTNER et al.: "Monoclonal antibodies detect P-glycoprotein"**

(73) Proprietor: **THE ONTARIO CANCER INSTITUTE
500 Sherbourne Street
Toronto Ontario M4X 1K9(CA)**

(72) Inventor: **Ling, Victor
35 Chester Hill Road
Toronto Ontario(CA)**
Inventor: **Kartner, Norbert
14 Crawford Drive
Ajax Ontario(CA)**

(74) Representative: **Crisp, David Norman et al
D. YOUNG & CO. 10 Staple Inn
London, WC1V 7RD(GB)**

EP 0 190 049 B1

CHEMICAL ABSTRACTS, vol. 101, no. 19, 5th November 1984, page 507, no. 168735w, Columbus, Ohio, US; C.J.O'HARA: "Characterization of monoclonal antibodies demonstrating specificity for drug-resistant tumor cells" & DISS. ABSTR. INT. B 1984, 45(2), 443

CHEMICAL ABSTRACTS, vol. 97, no. 17, 25th October 1982, page 512, no. 142881q, Columbus, Ohio, US; C.J.O'HARA et al.: "A monoclonal antibody demonstrating specificity for drug-resistant cells" & IMMUNOL. LETT. 1982, 5(1), 15-18

BIOLOGICAL ABSTRACTS, vol. 80, no. 7, 1985, page AB-594, no. 60303, Phildelphia, PA., US; D.R.BELL et al.: "Detection of P-glycoprotein in ovarian cancer: A molecular marker associated with multidrug resistance" & J. CLIN. ONCOL. 3(3): 311-315. 1985

NATURE, vol. 316, 29th August 1985, page 820; N.KARTNER et al.: "Detection of P-glycoprotein in multidrug-resistant cell lines by monoclonal antibodies"

## Description

The present invention relates to a method for selecting hybridomas (i.e. immortal clones) which produce antibodies specific to domains of a surface antigen which is usually not accessible at the surface of intact cells, and in particular to the P-glycoprotein surface antigen correlated with multidrug resistance (Juliano, R.L. & Ling, V. Biochem. Biophys. Acta 455, 152-162 (1976)). The invention also relates to such hybridomas and the monoclonal antibodies they produce.

Clinical resistance to combination chemotherapy poses a major obstacle to the successful treatment of neoplastic disease such as cancer. The underlying mechanisms of such clinical resistance are largely unknown. No diagnostic reagents are currently available to define the nature and degree of resistance in a patient, and thereby facilitate a rational choice of treatment, or provide a basis for more accurate prognosis. Numerous investigators have developed mammalian tissue culture and in vivo models of tumours resistant to diverse drugs in the hope of gaining insights into the mechanisms of clinical resistance to combination chemotherapy. Significantly, these models have a number of features in common which define what is now termed the multidrug resistance phenotype (Kartner, N., Riordan, J.R. & Ling, V. Science 221, 1285-1288 (1983); and Ling, V., Gerlach, J. & Kartner, N. Breast Cancer Res. Treat. 4, 89-94 (1984)). These features include (i) a pleiotropic cross-resistance to unrelated drugs, (ii) reduced net accumulation of the drugs involved, and (iii) the anomalous expression of a 170,000 dalton cell surface component, the P-glycoprotein. Other observations have also been noted in different cell lines, but qualitatively only the above features of multidrug resistance have remained consistent regardless of the initial drug of selection, or the mammalian species under investigation.

The striking correlation between P-glycoprotein expression and the expression of the multidrug resistance phenotype raises the possibility that P-glycoprotein might serve as a diagnostic marker for the detection of multidrug-resistant malignant cells in cancer patients. Also, because the P-glycoprotein is found in different mammalian species, it probably plays an important functional role in the mammalian cell surface. What this role might be in multidrug-resistant cells, or in normal tissue, is not yet understood.

We have now found it possible to obtain monoclonal antibodies that would serve as versatile analytical reagents, preferably recognizing homologous regions of the P-glycoproteins of different species. For this reason, in designing a strategy for selecting monoclonal antibodies specific for P-glyoprotein, several theoretical considerations were taken into account. Firstly, P-glycoprotein bears a carbohydrate moiety which may account for 20-30% of its molecular size (Ling, V., Kartner, N., Sudo, T., Siminovitch, L. & Riordan, J.R. Cancer Treat. Rep. 67, 869-874 (1983)). The extracellular domain of the polypeptide moiety appears to be either very limited, or sequestered by the relatively large carbohydrate moiety. Thus, conserved antigenic sites of P-glycoprotein, which are presumably associated with the polypeptide moiety, may not be accessible on the cell surface. Secondly, in other systems, selections based on binding to the cell surface have often yielded antibodies directed toward carbohydrate determinants of glycoconjugates. Such determinants vary with stage of differentiation and oncogenic transformation, and they may be common to different glycoproteins and glycolipids. Furthermore, the changes that occur in their distribution in the unstable environment of the neoplastic cell remain unclear. Thus, antibodies directed toward carbohydrate determinants of P-glycoprotein, or other glycoprotein antigens, are of limited utility in defining the gene product to which the antigenic sites are attached. Thirdly, since there appears to be a basal level of P-glycoprotein expression in drug-sensitive cells, P-glycoprotein may be a normal component of the mammalian cell surface. It follows that there may be regulation against an immune response to the extracellular domain of P-glycoprotein if it is recognized as a self antigen. Fourthly, it has been observed that monoclonal antibodies toward native proteins are often poor reagents for immunoprecipitation and immunoblotting analysis. This is a particularly important problem when considering membrane components, which are often denatured during analytical procedures. It would be difficult to determine precisely the identity of the antigen to which an antibody bears specificity, without the advantages of immunoprecipitation or immunoblotting analysis. Finally, we were aware that although attempts have been made to prepare P-glycoprotein specific monoclonal antibodies, using cell surface binding as a criterion for selection, these have failed, so far, to produce generally useful reagents proven to be directed against P-glycoprotein (O'Hara, C.J. & Price, G.B. Immunol. Lett. 5, 15-18 (1982); and Sugimoto, Y., Suzuki, H. & Tanaka, N. Biochem. Biophys. Res. Comm. 114, 969-975 (1983)).

Because of these theoretical considerations, a screening method for immortal clones (hereinafter also referred to as hybridomas) was designed that would avoid relying on a cell surface binding assay, and that would guarantee the selection of monoclonal antibodies which would serve as useful reagents for immunoblotting.

According to one aspect of the present invention there is provided a method for preparing a monoclonal antibody specific for a conserved region located in the C-terminal portion of the P-glycoprotein cell surface antigen correlated with multidrug resistance in mammalian species; said antibody having cross-reactivity with P-glycoprotein cell surface antigens of different mammalian species; and said antibody being further defined by its ability to bind isolated plasma membranes of multidrug resistant cells and its inability to bind live cells, comprising:

(1) immunizing a suitable mammal with cell or tissue fractions containing denatured plasma membranes of mammalian cells displaying multidrug resistance;

(2) immortalizing antibody producing cells from said immunized mammal to form a plurality of immortal antibody producing clones;

(3) differential screening of said clones for production of antibodies binding specifically to antigens present in isolated membranes of multidrug resistant cells but not in drug sensitive cells and which do not bind to multidrug resistant living cells; and

(4) said differential screening being done against membrane fractions of drug resistant and drug sensitive cells immobilized on a solid substrate.

According to another aspect of the present invention therefore there is provided a monoclonal antibody specific for a conserved region located in the C-terminal portion of the P-glycoprotein cell surface antigen correlated with multidrug resistance in mammalian species; said antibody having cross-reactivity with P-glycoprotein cell surgace antigens of different mammalian species; and said antibody being further defined by its ability to bind isolated plasma membranes of multidrug resistant cells and its inability to bind live cells.

Accordingly, the invention provides a method for selecting immortal clones which produce antibodies specific to domains of a cell surface antigen which is usually not accessible at the surface of intact cells, comprising the steps of: immunizing a suitable mammal with cell or tissue fractions containing plasma membranes of a mammalian cell line of interest; immortalizing antibody producing cells from said immunized mammal to form a plurality of immortal antibody producing clones; and screening said clones for production of antibodies specific against said antigen immobilized on a solid substrate.

The invention also provides hybridomas which produce antibodies, i.e. monoclonal antibodies, specific for such cell surface antigens, and particularly P-glycoprotein, as well as such monoclonal antibodies themselves when such hybridomas and monoclonal antibodies are produced by the aforesaid method of the invention.

A preferred embodiment of the invention will be described by way of non-limiting example with reference to the drawings in which:

Figure 1 shows examples of dot immunoblots used in the screening of hybridomas made in accordance to the invention;

Figure 2 shows in panel A, dot immunoblots using antibodies from eight cloned hybridomas against plasma membranes from both drug sensitive and resistant cells, and panel B shows monoclonal antibody staining of plasma membranes of drug resistant and sensitive cells;

Figure 3 shows graphic analyses of direct competitive binding assay of eight monoclonal antibodies against purified, radioiodinated antibody;

Figure 4 shows in panel A, Western blots of plasma membranes of drug sensitive and resistant cells using monoclonal antibody; in panel B indirect immunofluorescence staining of drug resistant cell with monoclonal antibody; and in panel C flow cytometry of indirect immunofluorescence staining using cell lines of different drug resistance; and

Figure 5 shows probing of pCHP1 lacZ fusion product containing a P-glycoprotein peptide fragment with different monoclonal antibodies.

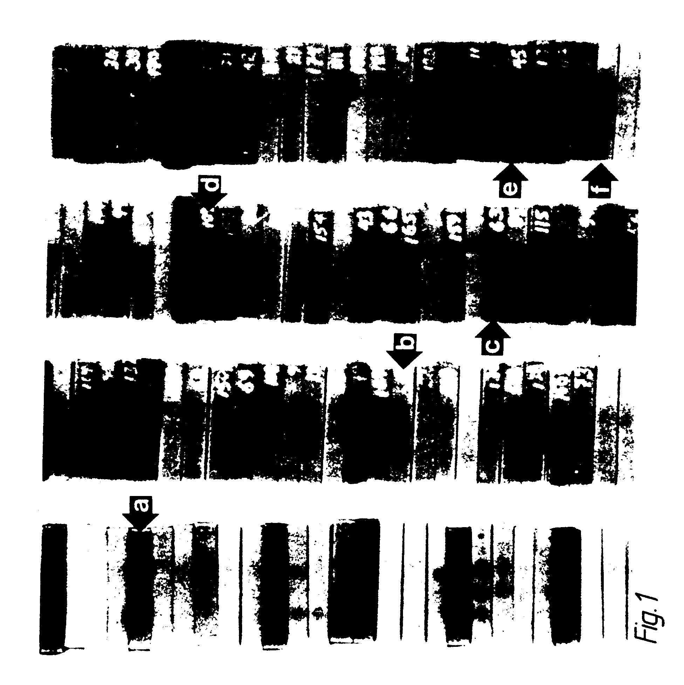

In the preferred method of the invention, mice were immunized with sodium dodecyl sulphate (SDS)-solubilized plasma membranes of multidrug-resistant Chinese hamster ovary (CHO) and human cell lines. Hybridomas were formed from spleen cells taken from the mice by fusion with a suitable fusion partner (see Example 1 below). Using a cross-species panel of detergent-solubilized plasma membranes immobilized on nitrocellulose filter test strips, it was ensured that the monoclonal antibodies selected would bind to conserved, drug resistance-specific epitopes in the denatured antigen, as they would be presented in protein immunoblots (Western blots) after SDS-polyacrylamide gel electrophoresis (SDS-PAGE). Results of a hybridoma screening, using nitrocellulose test strips, are shown in Fig. 1. As expected, a number of different specificities were observed, including those toward common mammalian, CHO or human antigens (Fig. 1: f, e and c, respectively). More importantly, specificities toward membranes of resistant cell lines, either CHO or human, or both, were observed (Fig. 1: b, d and a, respectively). The majority of strips are negative or only weakly stained. Specificities other than those indicated in Fig. 1 were only rarely seen and generally did not breed true. Primary screening of hybridoma supernatants of 1,007 wells which were positive for growth yielded 71 wells positive for drug resistant cell membrane (19 against human only, 37 against CHO only, an 15 against both). After expansion of these wells and secondary screening, 21 strongly positive wells remained (2 against human only, 5 against CHO only, and 14 against both). Hybridomas specific for drug resistant membranes were cloned twice by limiting dilution, using the above screening method to select specific antibody-producing clones. Eight stable clones were ultimately isolated (3 against CHO only, and 5 against both CHO and human). Losses of specific antibody secreting hybrids were presumably due to their genetic instability. The primary screening results probably reflect the presence of an initial population of unstable specific antibody-producing hybrids, rather than a large proportion of false positives. All further characterization was accomplished using the final, twice-cloned hybridomas.

Characterization of Monoclonal Antibodies

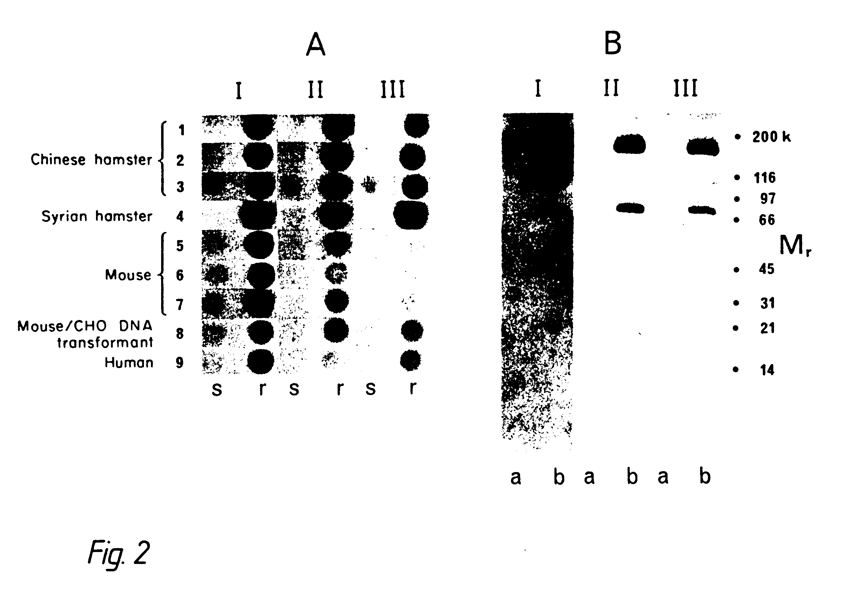

The results of the initial screening of the hybridomas clearly indicate that some differences in the binding sites recognized by the different antibodies are to be expected. For example, three of the eight final clones showed no affinity for membranes of drug-resistant human cells. In order to characterize these differences more precisely, supernatants from cloned hybridomas were screened with a larger panel of drug-sensitive and resistant plasma membranes. Test strips were also prepared for immuno-blotting plasma membranes of sensitive and resistant CHO cells after SDS-PAGE. Figure 2 (A and B) shows the results of these experiments. In Fig. 2(A) the results of dot blotting a larger panel of cell lines allows the classification of the eight monoclonal antibodies into three distinct groups (I, II and III) according to the pattern of staining obtained. No significant staining of drug-sensitive cell membranes was observed for any cell line, with any of the eight monoclonal antibodies. The monoclonal antibodies in Group I appear to stain all drug-resistant membranes tested. Those of Group II stain drug-resistant CHO and mouse membranes, but stain human membranes only very poorly. The single member of Group III stains drug-resistant CHO and human membranes, but not those of mouse. Other minor differences are also observed among the three groups.

In order to determine the molecular sizes of the antigens being stained by the monoclonal antibodies, Western blots of drug-sensitive and drug-resistant plasma membranes were overlayed with hybridoma supernatants and developed using a radiolabelled second antibody. As shown in Fig. 2-(B), all of the antibodies stained a major drug-resistance specific band of approximately 170,000 daltons. It is of interest that faint bands of lower molecular weight are also stained. These bands are variable among different preparations of plasma membranes from the same cells, and may be proteolytic fragments of the major staining band. None of these minor bands are observed in membranes of drug-sensitive cells. Similar putative breakdown products have been described previously, when using polyclonal rabbit antisera specific for P-glycoprotein of multidrug-resistant plasma membranes (Kartner, N., Riordan, J.R. & Ling, V. Science 221, 1285-1288 (1983)). Activation of membrane-associated proteases during detergent solubilization, or chemical cleavage of proteins during heating in the presence of SDS are common phenomena. Niman and Elder, for example, have described in detail the natural breakdown fragments of the murine retroviral envelope glycoprotein, gp70, and have used these fragments in a general scheme for mapping the antigenic sites of gp70 (Niman, H.L. & Elder, J.H. in Monoclonal Antibodies and T Cell Products (ed Katz, D.H.) 23-51 (CRC Press, Boca Raton, Florida, 1982)). In this context, it is of interest that when a single plasma membrane preparation was probed with different monoclonal antibodies, two different patterns of low molecular weight bands were seen. Fig. 2(B) shows the patterns obtained with representative antibodies of Groups I, II and III. Group I antibodies yielded one pattern, whereas Groups II and III yielded a different pattern. This suggests that a natural proteolytic cleavage site may separate the Group I epitope from the Groups II and III epitopes. Any attempt at mapping the epitopes more precisely, without additional information, may be complicated by the possibility that epitopes could be repeated within the P-glycoprotein polypeptide, or could be present on different members of a family of similar, but not identical P-glycoproteins.

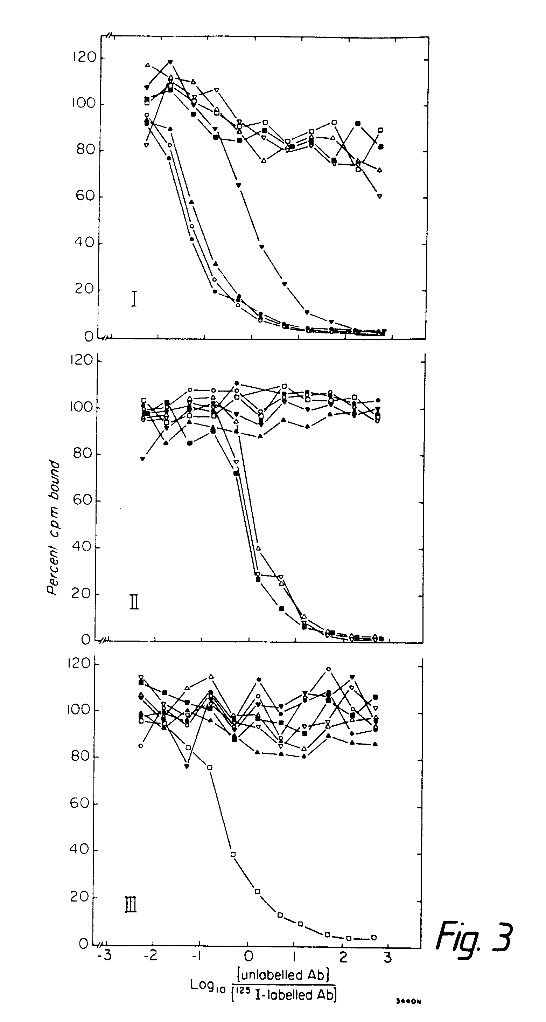

Differences in the peptide maps and in the staining patterns against a panel of different plasma membranes suggest that there may be three different antigenic sites. To provide more direct evidence of differences among the eight monoclonal antibodies and to determine the spatial relationships of their binding sites, competitive binding studies were performed with ascites of the different hybridomas and purified, radio labelled representatives of the three antibody groups. Typical competition assay results are shown in Fig. 3. These results clearly allow the classification of the antibodies into three groups (I, II and III) on the basis of their binding to three spatially distinct epitopes.

In the direct competitive binding assay of eight monoclonal antibodies against purified, radioiodinated antibody shown in Fig. 3, the target antigen was plasma membrane vesicles from $CH^R B30$, immobilized on nitrocellulose in 96-well plates. The abscissa in Fig. 3 is logarithmic scale of concentration ratio of unlabelled competing antibody to radiolabelled antibody. The ordinate is percent scale of cpm bound, normalized to cpm bound in the absence of competing antibody. Results of single simultaneous experiments are shown for clarity. Panel I shows competition of a labelled Group I antibody (C219) with dilutions of ascites fluids containing antibodies generated by the eight different hybridomas. No Group II or Group III antibodies compete with C219. The shift in competition curves of other Group I antibodies with respect to the self-competition curve of C219 probably reflects differences in binding avidity. Errors in quantitation of antibodies of different isotypes may also contribute to an apparent shift. Panel II shows competition of Group II antibody (C32) with other ascites fluids. Group I and III antibodies do not compete with C32. Binding kinetics of the three Group II antibodies appear to be very similar. Panel III displays the competition data for the single representative of Group III (C494). No Group I or Group II antibodies compete with C494. Antibodies tested were C11,O; C26,●; C32,△; C36,▲; C103,▽; C219, ▼; C494,□; and C699,■.

Specific Detection of P-Glycoprotein

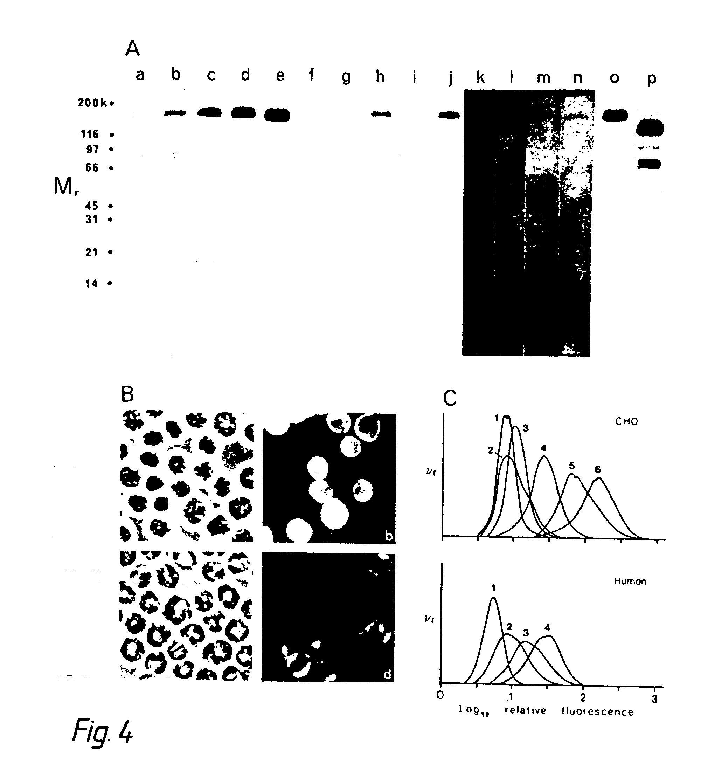

The evidence presented above strongly suggests that the monoclonal antibodies under investigation bind the P-glycoprotein of multidrug resistant cells, but this conclusion needs to be corroborated by testing the antibodies on cell lines which have been used previously to define the P-glycoprotein. Although dot blots have shown differential staining of drug-resistant cell membranes, it is not clear that the same antigen is bound in each case. Previous work has shown that homologous P-glycoproteins of consistent molecular weight are expressed in all multidrug resistant cell lines so far tested (Kartner et al. Science, supra,; Giavazzi, R., Kartner, N. & Hart, I.R. Cancer Chemother. Pharmacol. 13, 145-147 (1984); Kartner, N., Shales, M., Riordan, J.R. & Ling, V. Cancer Res. 43, 4413-4419 (1983)). Thus, Western blots of a series of plasma membranes were stained, using radio-iodinated monoclonal antibody. Fig. 4(A) shows the results of staining a series of plasma membranes from CHO cells of increasing colchicine resistance, and a drug-sensitive revertant (lanes a-d, and f). A daunorubicin resistant CHO cell line was also tested (lane e). Furthermore, blots of colchicine resistant mouse (lane h) and vinblastine resistant human cells (lane j) were probed (compare with drug sensitive parental cells, lanes g and i, respectively). Longer exposure of the blots (lanes k-n) reveals faint bands in the membranes of sensitive parents and revertant cells in lanes a, f, g and i respectively. Low level expression of P-glycoprotein in drug-sensitive cells has been reported previously (Kartner et al. Science, supra). Trivial explanations for the apparent homologies of the 170k bands in different species, such as detection of phosphate or common carbohydrate epitopes are ruled out by the controls in lanes o and p. The correlation of increasing P-glycoprotein expression with increasing multidrug resistance and the conservation of the antigen molecular size regardless of the drug of selection in a number of mammalian species confirm that the antigen bound by the monoclonal antibodies is P-glycoprotein as it has been previously defined (Kartner et al. Science, supra).

The foregoing demonstrates that detergent-solubilized and denatured P-glycoprotein immobilized on nitrocellulose filters is detected with the monoclonal antibodies under study. In many applications for diagnostic or research purposes it is important that an antibody be capable of detecting the antigenic site as it is presented in intact cells and tissues. In this regard it has been found, using both radiolabelled antibody and indirect, immunofluorescence staining, that none of the monoclonal antibodies binds the surface of intact multidrug resistant cells. Nonetheless, as is seen in Fig. 4(B), using fixed and permeabilized cells, the monoclonal antibodies are capable of labelling the surface membrane of resistant CHO and human cells. It is apparent from this work that the epitopes defined by the monoclonal antibodies are either cryptic, or exposed on the cytoplasmic face of the plasma membrane. When native plasma membrane vesicles are spotted onto nitrocellulose, all eight antibodies bind to them at least as well as to SDS solubilized membranes, showing that protein unfolding is probably not a requisite for epitope recognition. It is presumed therefore, that the antibodies bind the exposed cytoplasmic domain of relatively native P-glycoprotein in disrupted, or inside-out membrane vesicles. Fig. 4(C) demonstrates that with the use of flow cytometry the degree of staining of cells with these monoclonal antibodies can be determined semi-quantitatively and correlates with the degree of drug resistance of the cells. Such a relationship exists between the degree of drug resistance and the amount of P-glycoprotein expressed in drug-resistant cells, (Kartner et al. Science, supra; Kartner et at. Cancer Res., supra, Giavazzi et al. Cancer Chemother Pharmacal, supra; Riordan, J.R., and Ling, V. J. Biol. Chem. 254, 12701-12705 (1979)).

This same correlation, which is obtained between fluorescence intensity and the degree of drug resistance of the resistant cells shown in Fig. 4(C), is strong corroborative evidence that P-glycoprotein is being stained specifically. Furthermore, Fig. 4(B and C) demonstrates that these monoclonal antibodies are indeed useful as reagents for the staining of cells and tissues. In addition to their use in indirect immunofluorescence methods, the antibodies have been used in conventional immunoperoxidase staining techniques.

## LOCALIZATION OF THREE EPITOPES TO A CONSERVED DOMAIN OF P-GLYCOPROTEIN

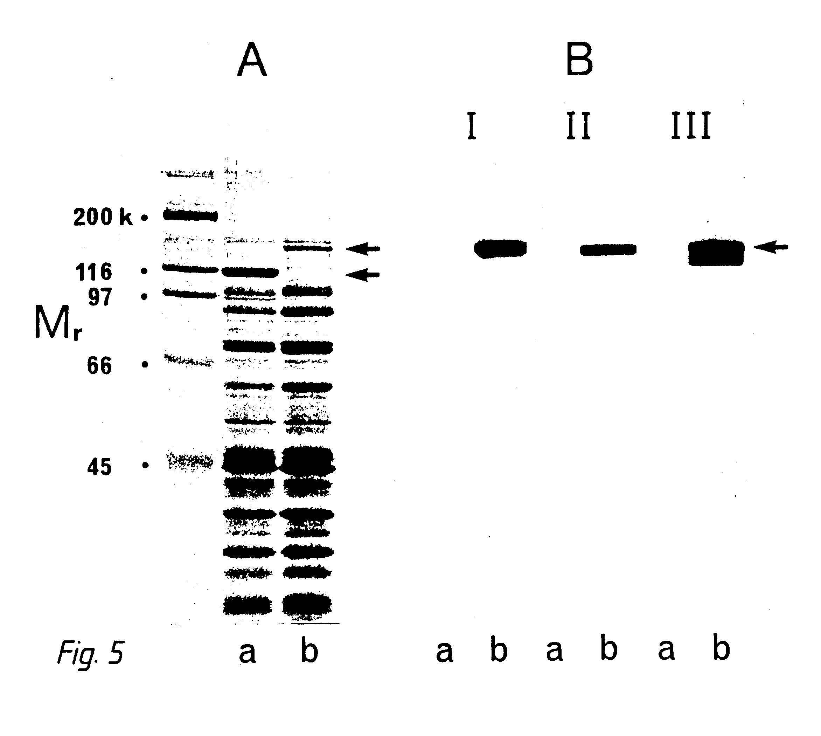

The Group I monoclonal antibody, C219, has been used to obtain a 700 bp cDNA probe, pCHP1, from a λgt11 expression library of the multidrug-resistant CHO cell line, CH$^R$B30. The lacZ fusion product of pCHP1 presumably includes the epitope which is bound by C219. Independent evidence, including resistance associated gene amplification in Southern blots of genomic DNA probed with pCHP1, strongly supports the notion that the cDNA codes for a fragment of P-glycoprotein, presumably in the region of its C-terminus. For a number of reasons, it is of interest to know whether the antibodies recognizing different epitopes bind the same lacZ fusion product. Firstly, the existence of more than one epitope on the pCHP1-coded polypeptide would provide very strong corroborative evidence that the cDNA does indeed encode a fragment of the P-glycoprotein. Although a single P-glycoprotein specific monoclonal antibody could possibly bind a non-P-glycoprotein antigen, it is unlikely that antibodies binding independent epitopes would bind the same non-P-glycoprotein antigen. Secondly, peptide fragments such as that encoded by pCHP1 could serve as reagents for determining additional subgroups among the groups of monoclonal antibodies that bind to the lacZ fusion product. Finally, binding data would allow a crude localization of the different epitopes, possibly providing some insights into the structure of the P-glycoprotein. In Figure 5(A) lysates of control λgt11-infected bacteria and pCHP1-infected bacteria have been resolved by SDS-PAGE. In the control lysate (lane a) a heavy B-galactosidase band is seen. In the pCHP1 lysate this band has disappeared and a major lacZ fusion band is seen at about 145,000 daltons. This band is approximately of the size expected if most, or all, of the pCHP1 insert encodes an open reading frame. Numerous new bands of somewhat lower molecular weight than the major lacZ fusion band are also seen. These are presumably proteolytic fragments of the lacZ fusion product. Figure 5(B) shows the results of probing Western blots of the lacZ fusion product of pCHP1 with monoclonal antibodies of each of the three groups. All three epitopes are detected in the same polypeptide fragment. Although the Groups I and II antibodies appear to stain only the major lacZ fusion product band, the Group III antibody stains many of the lower molecular weight fragments as well. Since the lower molecular weight bands are all larger than B-galactosidase, and the insert-encoded peptide is attached to the C-terminus of B-galactosidase, crude mapping of the epitopes is possible. The staining of the putative proteolytic fragments suggests that the Group III epitope is distal from the C-terminus of the pCHP1 encoded peptide. Since only the major, or largest, bands appear to be stained by the Groups I and II antibodies, it is likely that they are clustered near the C-terminus of the peptide. Dot blots of pCHP1 lysates and control λgt11 lysates confirm that all eight monoclonal antibodies bind the lacZ fusion product. This is a surprising result, since pCHP1 probably encodes less than 20% of the P-glycoprotein polypeptide. The presence of three independent epitopes in the lacZ fusion product clearly confirms that pCHP1 codes for P-glycoprotein. Furthermore, this observation suggests than an immunogenic and conserved domain exists in the C-terminal region of the P-glycoprotein.

In summary, a strategy has been designed and employed to select for monoclonal antibodies that bind conserved, multidrug resistance-associated antigens after SDS denaturation and immobilization on nitrocellulose. The method of hybridoma screening used in the present work is a generally useful technique for producing monoclonal antibodies against cell surface antigens. It assures reagents that will be useful for identifying antigens in the powerful Western blotting technique. Furthermore, it avoids some of the possible problems associated with cell surface binding assays and, thus, provides a complementary alternative for hybridoma screening. The method also provides a means of generating antibodies to cytoplasmic domains of membrane antigens. These domains are of great interest because of their functional association with the cytoplasm of the cell, and they have, as yet, rarely been characterized in detail.

In the production of monoclonal antibodies that differentially bind plasma membranes of multidrug resistance cells, eight different antibodies were obtained. It is significant that all eight antibodies show specificity toward the same antigen, the P-glycoprotein, supporting the notion that the expression of P-glycoprotein is the major alteration of the multidrug-resistant cell surface. Furthermore, each of the eight antibodies binds one of three distinct epitopes confined to a region less than 240 amino acids in length. This domain is apparently accessible only on the cytoplasmic surface of the plasma membrane and is presumably in the C-terminal region of the P-glycoprotein polypeptide. It is interesting that three independent antigenic sites are clustered in this region. This suggests that the domain is relatively more immunogenic than the majority of the protein. Possibly this region represents a hydrophilic domain in an otherwise relatively hydrophobic protein. Alternatively, it has recently been suggested that regions of a polypeptide that are conformationally more fluid are more likely to be antigenic. Thus, the termini of polypeptides are often relatively antigenic regions. It is also of interest that no antibodies that bind an extracellular domain of the P-glycoprotein were obtained. The carbohydrate moiety, in particular, should be relatively unaffected by detergent solubilization and, for other glycoproteins, this moiety often accounts for a high proportion of antigenic sites. One explanation for the present observation is that carbohydrate determinants of P-glycoprotein might be common to other glyconjugates, present in both drug-sensitive and drug resistant cells, and would not be detected in a differential screening assay. Alternatively, recognition of the extracellular domain of P-glycoprotein as a self-antigen, particularly if this domain is structurally conserved among mammalian species, could account for this observation.

The epitopes which have been defined in the cytoplasmic domain of the P-glycoprotein do not appear to be equally represented in different mammalian species. One of the epitopes described (Group I) is detected equally well in rodent and human P-glycoproteins and, therefore, appears to be highly conserved. The Group II epitope is detected in hamster, less well in mouse, and very poorly in human P-glycoprotein. This observation may reflect a species related structural divergence in this region of the protein. On the other hand, the Group III epitope is detected in hamster and less well in human P-glycoprotein, but very poorly in mouse. This epitope could represent a more variable region of P-glycoprotein structure. Another explanation might be that different members of a P-glycoprotein multigene family are expressed in different cell lines. This possibility as been previously proposed as a possible mechanism for variation in the cross-resistance patterns of multidrug resistant cells selected from common parental lines. The particular drug of selection, and possibly the characteristics of the parental cell line, may affect the amplification and overexpression of specific genes, or specific combinations of genes. Recently, Southern blot analysis of genomic DNA with a P-glycoprotein specific cDNA probe (pCHP1) has provided strong evidence in support of a multigene family of P-glycoproteins.

The antibodies described in the present work are the first molecular probes available for the assessment of the role of P-glycoprotein in human tumours and for the elucidation of the molecular biology of the multidrug resistance phenotype. Clinical application of the monoclonal antibodies for diagnosis of therapy-resistant disease is an obvious utility for this invention. Early detection of elevated levels of P-glycoprotein in tumours might indicate a departure from routine chemotherapy an application of novel treatments that may circumvent the multidrug resistance phenotype. Preliminary findings of elevated amounts of P-glycoprotein in some ovarian carcinomas of patients failing chemotherapy demonstrate the utility of the present monoclonal antibodies for this purpose. Furthermore, these reagents will be useful in the sensitive and specific detection of P-glycoprotein in experimental systems and in normal mammalian tissues. This will allow the study of the biosynthesis and functional role of P-glycoprotein in drug-resistant cells. Such studies will provide insights into structure and function at the mammalian cell surface, and also will establish a theoretical basis that might lead to improved chemotherapy of presently nonresponsive neoplastic disease.

Example 1

Twelve week old (BALB/c x C3H)F1 mice were immunized with injections of purified plasma membranes isolated according to Riordan and Ling (Riordan, J.R. & Ling, V. J. Biol. Chem. 254, 12701-12705 (1979)) from $CH^RB30$ and $CEM/VLB_{500}$ cell lines. Membrane protein was assayed according to a modification (Peterson, G.L. Anal. Biochem. 83, 346-356 (1977)) of the method of Lowry et al (Lowry, O.H., Rosebrough, N.J., Farr, A.L. & Randall, R.J. J. Biol. Chem. 193, 265-275 (1951)). Membranes were solubilized in a 2% SDS-containing buffer as for SDS-PAGE, with a protein:SDS ratio of 1:1.4, and were emulsified 1:1 with Freund's incomplete adjuvant. The first injection consisted of 200 $\mu$g of $CH^RB30$ membrane intraperitoneally, followed after 14 days by a second, identical injection. After 14 months the mice were injected with a mixture of 100 $\mu$g each of $CH^R30$ and $CEM/VLB_{500}$ plasma membranes. Identical injections were given 14 and 21 days after the first injection in this series. Additionally on day 21 a mixture containing 100 $\mu$g each of sonicated plasma membrane vesicles of $CH^RB30$ and $CEM/VLB_{500}$ was injected intravenously. On day 26 spleens were removed aseptically and splenocytes fused 3:1 with the Sp2/0-Agl4 fusion partner (Shulman, M., Wilde, C.D. & Kohler, G. Nature 276, 269-270 (1978)). Cells were plated in 96-well plates at $10^5$ myeloma cells per well in RPMI-1640 medium containing 100 $\mu$M hypoxanthine, 0.5 $\mu$M aminopterin, 30 $\mu$M thymidine, 2 mM L-glutamine 50 $\mu$M 2-mercaptoethanol and 15% fetal bovine serum. When wells appeared about one-quarter confluent (typically 50-80% of wells after 10-14 days), they were screened for specific antibody producing hybrids. Test strips were prepared by

spotting 1 μl of SDS-solubilized plasma membrane (prepared as for SDS-PAGE) at 1 mg/ml concentration onto numbered 5 mm x 25 mm nitrocellulose strips (see Fig. 1, Panel A). The dried dot blots were blocked in 3% bovine serum albuin (BSA)-/saline for 1h at 37° and were placed into test tubes containing hybridoma supernatants diluted 1:10 in BSA/saline. After overnight incubation at 4 the strips were washed in several changes of PBS and were developed with $^{125}$I-labelled IgG goat anti-mouse immunoglobulins (Cappel; $10^6$ cpm/ml in 3% BSA/saline) for 6 h at 25°. After washing and drying, the test strips were taped to stiff plastic sheets and exposed overnight, with intensifying screens, on Kodak X-AR5 film at -70°. Typically about 1000 strips could be processed at the same time. Promising wells were expanded and cloned twice by limiting dilution. The above screening method was repeated to pick positive clones.

## Claims

1. A method for preparing a monoclonal antibody specific for a conserved region located in the C-terminal portion of the P-glycoprotein cell surface antigen correlated with multidrug resistance in mammalian species; said antibody having cross-reactivity with P-glycoprotein cell surface antigens of different mammalian species; and said antibody being further defined by its ability to bind isolated plasma membranes of multidrug resistant cells and its inability to bind live cells, comprising:
   (1) immunizing a suitable mammal with cell or tissue fractions containing denatured plasma membranes of mammalian cells displaying multidrug resistance;
   (2) immortalizing antibody producing cells from said immunized mammal to form a plurality of immortal antibody producing clones;
   (3) differential screening of said clones for production of antibodies binding specifically to antigens present in isolated membranes of multidrug resistant cells but not in drug sensitive cells and which do not bind to multidrug resistant living cells; and
   (4) said differential screening being done against membrane fractions of drug resistant and drug sensitive cells immobilized on a solid substrate.

2. A method as claimed in claim 1, wherein SDS denatured plasma membranes are used for immunizing a suitable mammal.

3. A method as claimed in either of claims 1 and 2, wherein the antibody is selected by screening for the hydridomas producing antibody specific against the P-glycoprotein antigen immobilized on a solid substrate.

4. A method as claimed in any preceding claim, wherein the antigen is immobilized on a nitrocellulose solid substrate.

5. A method as claimed in any preceding claim, wherein the clones are screened using detergent solubilized plasma membranes of the cell line of interest immobilized on nitrocellulose.

6. A method as claimed in any preceding claim, wherein the mammal being immunized is selected from mice, rats, hamsters and guinea pigs.

7. A method as claimed in any preceding claim wherein the mammal is immunized with cell or tissue fractions derived from more than one multi-drug resistant mammalian cell line.

8. A method as claimed in any preceding claim, wherein the cell or tissue fractions are derived from more than one mammalian species.

9. A monoclonal antibody specific for a conserved region located in the C-terminal portion of the P-glycoprotein cell surface antigen correlated with multidrug resistance in mammalian species; said antibody having cross-reactivity with P-glycoprotein cell surface antigens of different mammalian species; and said antibody being further defined by its ability to bind isolated plasma membranes of multidrug resistant cells and its inability to bind live cells.

10. A monoclonal antibody as claimed in claim 9, wherein the antibody is specific for a conserved region located in the C-terminal portion of the human P-glycoprotein.

## Revendications

1. Procédé pour la production d'un anticorps monoclonal spécifique d'une région préservée située dans la partie à l'extrémité C-terminale de l'antigène de surface cellulaire P-glycoprotéine mis en corrélation avec la polyrésistance à des médicaments dans une espèce mammalienne, ledit anticorps ayant une réactivité croisée avec des antigènes de surface cellulaire de type P-glycoprotéine de différentes espèces mammaliennes, et ledit anticorps étant en outre défini par son aptitude à se lier à des membranes cytoplasmiques isolées de cellules polyrésistantes à des médicaments et par son inaptitude à se lier à des cellules vivantes, comprenant:

(1) l'immunisation d'un mammifère approprié avec des fractions cellulaires ou tissulaires contenant des membranes cytoplasmiques dénaturées de cellules mammaliennes manifestant une polyrésistance à des médicaments;

(2) l'immortalisation de cellules produisant des anticorps, provenant dudit mammifère immunisé, pour la formation d'une pluralité de clones immortels producteurs d'anticorps;

(3) le criblage différentiel desdits clones pour la production d'anticorps se liant spécifiquement aux antigènes présents dans des membranes isolées de cellules polyrésistantes à des médicaments, mais non dans des cellules sensibles aux médicaments, et qui ne se lient pas à des cellules vivantes, polyrésistantes à des médicaments; et

(4) ledit criblage différentiel étant effectué contre des fractions membranaires de cellules résistantes à des médicaments et de cellules sensibles à des médicaments, immobilisées sur un support solide.

2. Procédé selon la revendication 1, dans lequel des membranes cytoplasmiques dénaturées au moyen de SDS sont utilisées pour l'immunisation d'un mammifère approprié.

3. Procédé selon la revendication 1 ou 2, dans lequel l'anticorps est sélectionné par criblage pour la détection d'hybridomes producteurs d'anticorps spécifique contre l'antigène P-glycoprotéine immobilisé sur un support solide.

4. Procédé selon l'une quelconque des revendications précédentes, dans lequel l'antigène est immobilisé sur un support solide en nitrocellulose.

5. Procédé selon l'une quelconque des revendications précédentes, dans lequel on crible les clones en utilisant des membranes cytoplasmiques, solubilisées au moyen d'un détergent, de la lignée cellulaire concernée, immobilisées sur nitrocellulose.

6. Procédé selon l'une quelconque des revendications précédentes, dans lequel le mammifère immunisé est choisi parmi la souris, le rat, le hamster et le cobaye.

7. Procédé selon l'une quelconque des revendications précédentes, dans lequel le mammifère est immunisé à l'aide de fractions de cellules ou de tissus provenant de plus d'une lignée cellulaire mammalienne polyrésistante à des médicaments.

8. Procédé selon l'une quelconque des revendications précédentes, dans lequel les fractions de cellules ou de tissus proviennent de plus d'une espèce mammalienne.

9. Anticorps monoclonal spécifique d'une région préservée située dans la partie à l'extrémité C-terminale de l'antigène de surface cellulaire P-glycoprotéine mis en corrélation avec la polyrésistance à des médicaments dans une espèce mammalienne, ledit anticorps ayant une réactivité croisée avec des antigènes de surface cellulaire de type P-glycoprotéine de différentes espèces mammaliennes, et ledit anticorps étant en outre défini par son aptitude à se lier à des membranes cytoplasmiques isolées de cellules polyrésistantes à des médicaments et par son inaptitude à se lier à des cellules vivantes.

10. Anticorps monoclonal selon la revendication 9, dans lequel l'anticorps est spécifique d'une région préservée située dans la partie à l'extrémité C-terminale de la P-glycoprotéine humaine.

**Patentansprüche**

1. Verfahren zum Herstellen eines monoklonalen Antikörpers, der für einen konservierten Bereich spezifisch ist, der im C-terminalen Teil des P-Glykoprotein-Zelloberflächen-Antigens liegt, das mit Multi-Arzneimittelresistenz in Säugetierarten korreliert ist; wobei der Antikörper mit P-Glykoprotein-Zelloberflächen-Antigenen von verschiedenen Säugetierarten kreuzreaktionsfähig ist; und wobei der Antikörper ferner definiert ist durch seine Fähigkeit, isolierte Plasmamembranen von multi-arzneimittelresistenten Zellen zu binden, und durch seine Unfähigkeit, lebende Zellen zu binden, wobei das Verfahren folgende Schritte aufweist:

(1) Immunisieren eines geeigneten Säugetiers mit Zell- oder Gewebsfraktionen, die denaturierte Plasmamembranen von Säugetierzellen, die eine Multi-Arzneimittelresistenz zeigen, enthalten;

(2) Immortalisieren von Antikörper-erzeugenden Zellen aus dem immunisierten Säugetier, um eine Vielzahl von unsterblichen Antikörper-erzeugenden Klonen zu bilden;

(3) differentielles Screening der genannten Klone zur Erzeugung von Antikörpern, die sich spezifisch an Antigene binden, die in isolierten Membranen von multi-arzneimittelresistenten Zellen, jedoch nicht in arzneimittelempfindlichen Zellen vorhanden sind, und die sich nicht an multi-arzneimittelresistente lebende Zellen binden; und

(4) Durchführen des differentiellen Screenings gegenüber Membranfraktionen von arzneimittelresistenten und arzneimittelempfindlichen Zellen, die auf einem Festsubstrat immobilisiert sind.

2. Verfahren nach Anspruch 1, wobei SDS-denaturierte Plasmamembranen zur Immunisierung eines geeigneten Säugetiers eingesetzt werden.

3. Verfahren nach einem der Ansprüche 1 und 2, wobei der Antikörper durch Screening bezüglich des Hybridome-erzeugenden Antikörpers ausgewählt wird, der gegen das auf einem Festsubstrat immobilisierte P-Glykoprotein-Antigen spezifisch ist.

4. Verfahren nach einem der vorhergehenden Ansprüche, wobei das Antigen auf einem Colloxylinum-Festsubstrat immobilisiert wird.

5. Verfahren nach einem der vorhergehenden Ansprüche, wobei das Screening der Klone unter Einsatz von mit Netzmittel löslich gemachten Plasmamembranen der interessierenden Zell-Linie, die auf Colloxylinum immobilisiert ist, erfolgt.

6. Verfahren nach einem der vorhergehenden Ansprüche, wobei das zu immunisierende Säugetier aus Mäusen, Ratten, Hamstern und Meerschweinchen ausgewählt wird.

7. Verfahren nach einem der vorhergehenden Ansprüche, wobei das Säugetier mit Zell- oder Gewebsfraktionen immunisiert wird, die von mehr als einer multi-arzneimittelresistenten Säugetier-Zell-Linie gewonnen sind.

8. Verfahren nach einem der vorhergehenden Ansprüche, wobei die Zell- oder Gewebsfraktionen von mehr als einer Säugetierart gewonnen sind.

9. Monoklonaler Antikörper, der für einen konservierten Bereich spezifisch ist, der im C-terminalen Teil des P-Glykoprotein-Zelloberflächen-Antigens liegt, das mit der Multi-Arzneimittelresistenz in Säugetierarten korreliert ist; wobei der Antikörper mit P-Glykoprotein-Zelloberflächen-Antigenen von verschiedenen Säugetierarten kreuzreaktionsfähig ist; und wobei der Antikörper ferner definiert ist durch seine Fähigkeit, isolierte Plasmamembranen von multi-arzneimittelresistenten Zellen zu binden, und durch seine Unfähigkeit, lebende Zellen zu binden.

10. Monoklonaler Antikörper nach Anspruch 9, wobei der Antikörper für einen konservierten Bereich spezifisch ist, der im C-terminalen Teil des Human-P-Glykoproteins liegt.

Fig.1

*Fig. 2*

EP 0 190 049 B1

Fig. 3

3440N

Fig. 4

*Fig. 5*